(19) Europäisches Patentamt
European Patent Office
Office européen des brevets

(11) Publication number : 0 282 241 B1

(12) # EUROPEAN PATENT SPECIFICATION

(45) Date of publication of patent specification : 14.08.91 Bulletin 91/33

(51) Int. Cl.[5] : C07F 7/18, C07D 205/08, C07F 7/08

(21) Application number : 88301908.5

(22) Date of filing : 04.03.88

(54) **Beta-lactam compounds and production process thereof.**

The file contains technical information submitted after the application was filed and not included in this specification

(30) Priority : 06.03.87 JP 52816/87

(43) Date of publication of application : 14.09.88 Bulletin 88/37

(45) Publication of the grant of the patent : 14.08.91 Bulletin 91/33

(84) Designated Contracting States : AT BE CH DE ES FR GB GR IT LI LU NL SE

(56) References cited :
EP-A- 0 171 064
EP-A- 0 224 059
JOURNAL OF THE CHEMICAL SOCIETY, no. 22, 1982, pages 1324-1325; M. SHIBASAKI et al.: "A simple preparation of (+)-4-phenylthioazetidin-2-one and an asymmetric synthesis of (+)-thienamycin"
TETRAHEDRON LETTERS, vol. 28, no. 17, 1987, pages 1857-1860, Pergamon Press; R. ROBINSON et al.: "The international journal for the rapid publication of preliminary communications in organic chemistry"

(73) Proprietor : Tamura, Yasumitsu
9-24 Sakasegawa 2-chome
Takarazuka Hyogo Pref., (JP)
Proprietor : SHIONOGI & CO., LTD.
1-8, Doshomachi 3-chome Chuo-ku
Osaka 541 (JP)

(72) Inventor : Tamura, Yasumitsu
9-24, Sakasegawa 2-chome
Takarazuka Hyogo 665 (JP)
Inventor : Nishi, Koichi
19-25-308 Takadono 2-chome
Asahi-ku Osaka 535 (JP)
Inventor : Haruta, Junichi
4-3754, Gotenyama-minami-machi
Hirakata Osaka 573 (JP)

(74) Representative : Burford, Anthony Frederick et al
W.H. Beck, Greener & Co. 7 Stone Buildings
Lincoln's Inn
London WC2A 3SZ (GB)

EP 0 282 241 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel β-lactam compounds, wich contain a hydroxyethyl group at the 3-position, having the hydroxyl group in the hydroxyethyl group protected or unprotected, and a butynyl group at the 4-position, and also to processes for producing the compounds.

Thienamycin (1) is produced by fungi of Actinomyces and is known to possess extremely potent antimicrobial activities. Its discovery initiated intensive research on carbapenem compounds, resulting in publication of imipenem (2) in 1981.

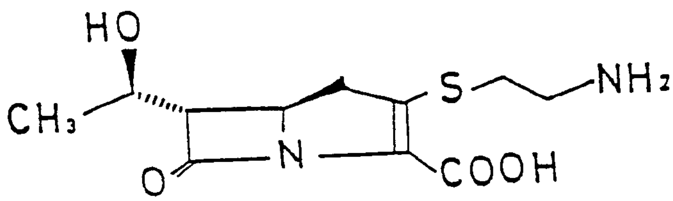

Thienamycin (1)

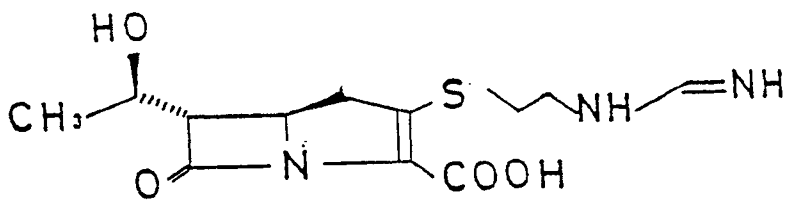

Imipenem (2)

However, these carbapenem compounds are far from satisfactory in terms of chemical stability and resistance to renal dehydropeptidase-I (DHP-I). Consequently, investigation has been conducted into more stable carbapenem compounds exhibiting potent antimicrobial activity, which led in 1984 to the finding that 1β-methylcarbapenem (3) having a β-methyl group at the 1-position is stable and possesses strong antimicrobial activity.

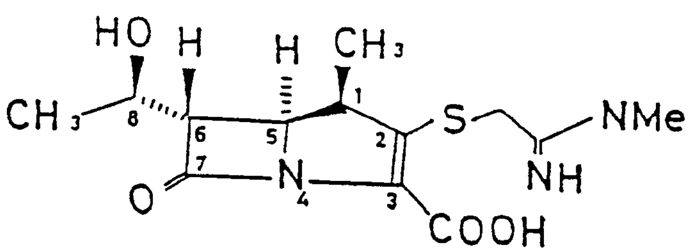

3

The said compound (3) contains four consecutive asymmetric carbons ($C_1$, $C_5$, $C_6$, $C_8$), and in view of the fact that most of the publications on its synthesis are concerned with the utilization of optically active azetidinone (4) as an intermediate, the synthesis of the intermediate (4) has been considered to be the key to the commercial production of the said compound (3).

According to the literature published so far on the synthesis of the compound (4), four reports dealt with the stereospecific synthesis, one report was concerned with the stereospecific reduction and one report with isomerization, as is illustrated below in the reaction formulae :

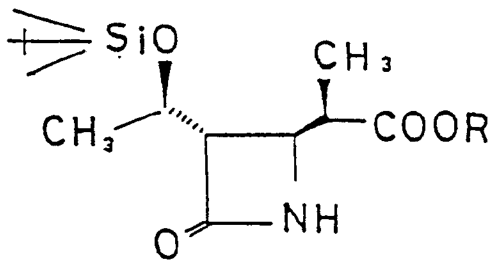

4

(Stereospecific synthesis)

1)

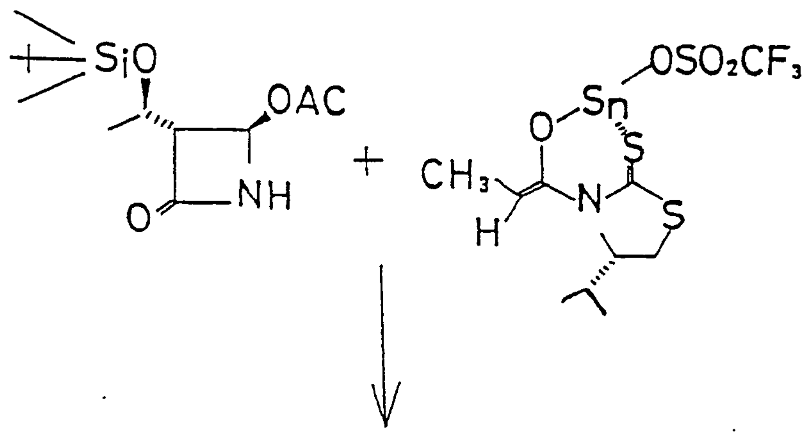

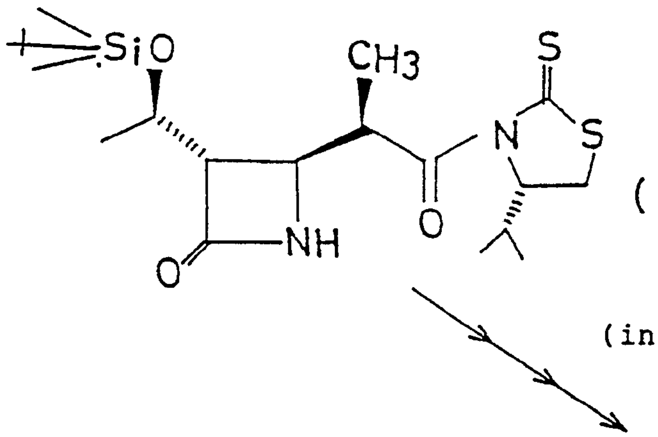

(α : β = 9 : 91)

74 %

(in three steps)

4

J.Am.Chem.Soc., 108, 4673 (1986)

2 )

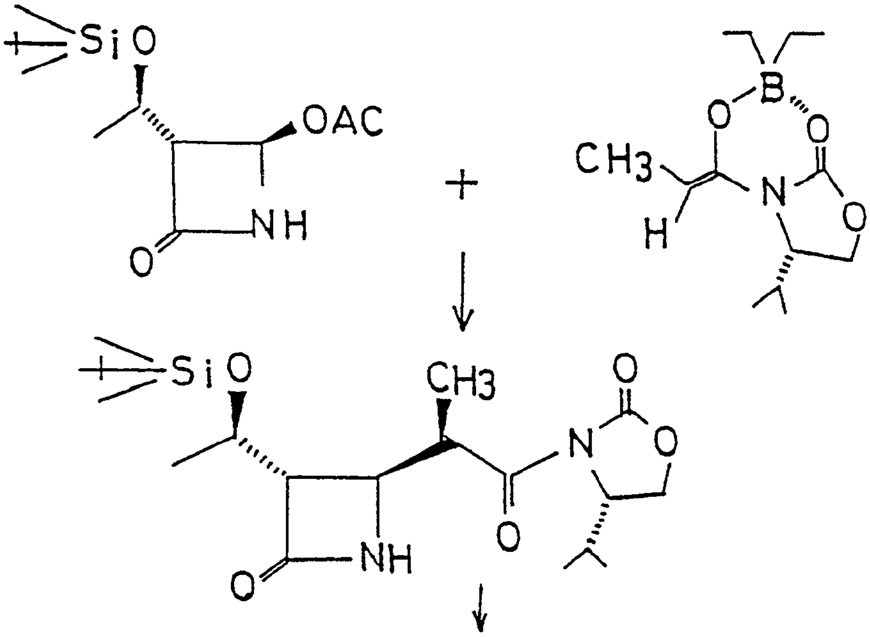

4

(α : β = 1 : 99)

73.1 %

J.Am.Chem.Soc., 108, 4675 (1986)

3 )

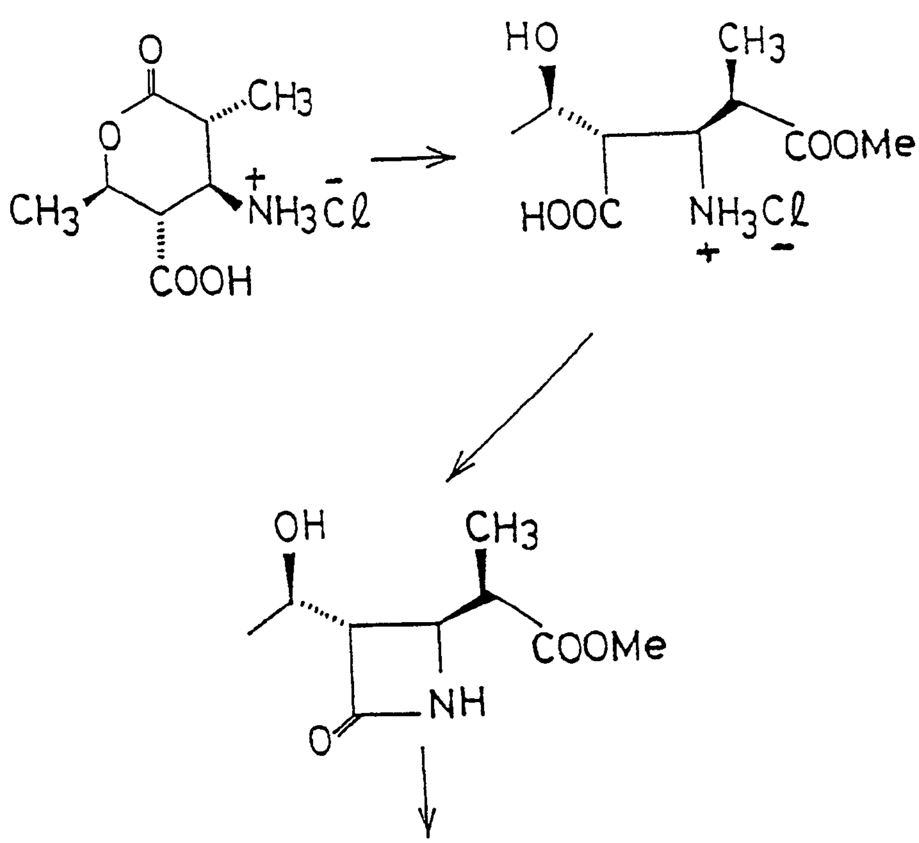

4 75 %

Tetrahedron Lett., 28, 83 (1987)

4 )

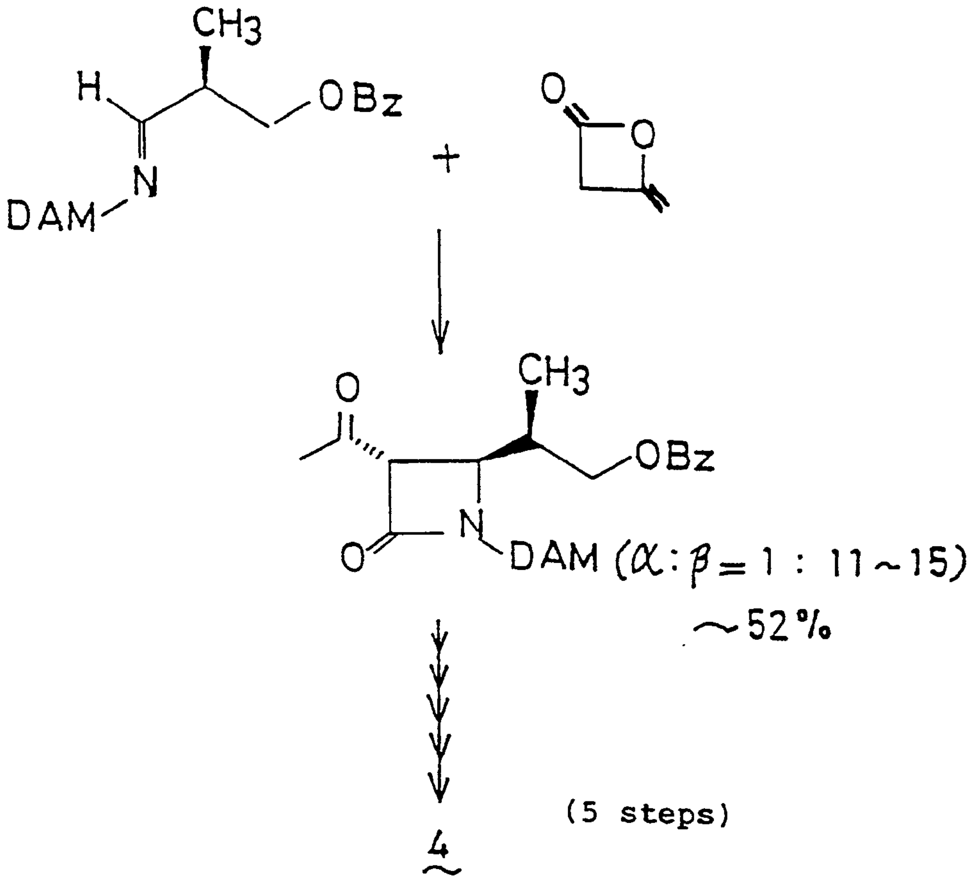


4

The 13th Symposium of Progresses of Reactions & Syntheses, pp.72 (1986)

(Stereospecific reduction)

5 )

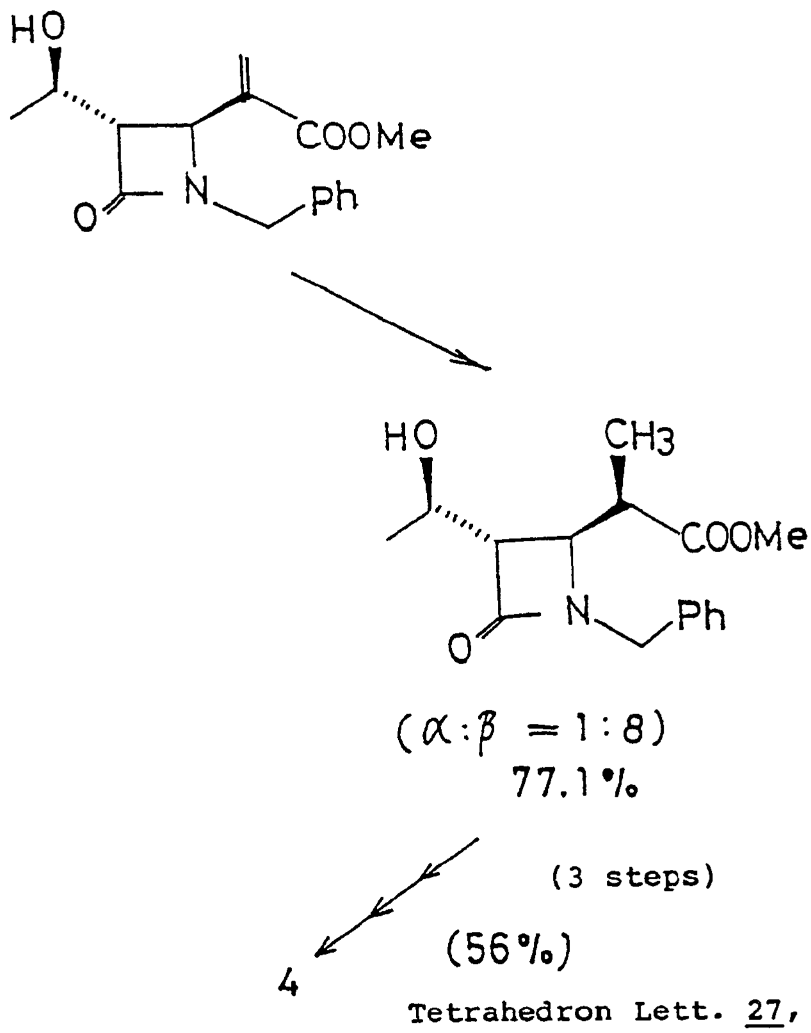


4

Tetrahedron Lett. 27, 2149 (1986)

[Isomerization]

6 )

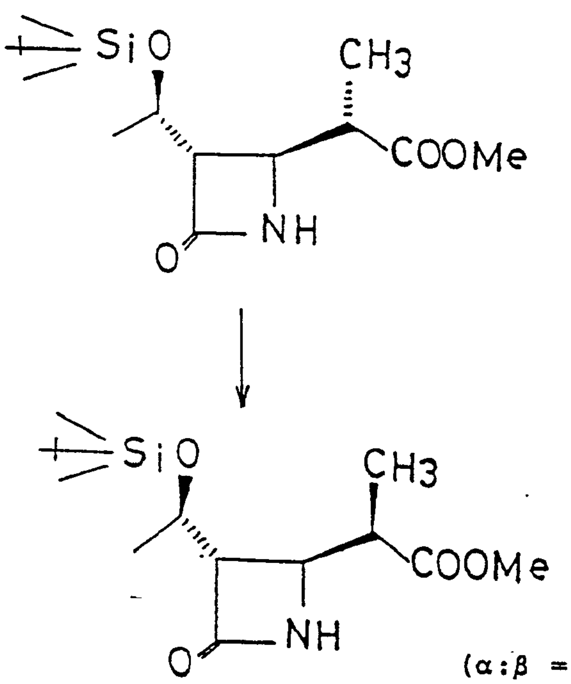

(α:β = 2:98)

Unknown yield

8th Symposium on Medicinal Chemistry, pp. 21 (1986)

EP-A-0171064 discloses the preparation of 3-(1-hydroxyethyl)-4-acetoxy-2-azetidinone from a lithium enolate of an alkyl 3-hydroxybutanoate and ethynylaldimine by the following steps :

(a) reaction of said enolate and ethynylaldimine to form 3-(1-hydroxyethyl)-4-ethynyl-2-azetidione ;

(b) reaction of said 3-(1-hydroxyethyl)-4-ethynyl-2-azetidinone with a mercuric compound in the presence of sulphuric acid and/or ammonium sulphate to form 3-(1-hydroxyethyl)-4-acetyl-2-azetidinone ; and

(c) reaction of said 3-(1-hydroxyethyl)-4-acetyl-2-azetidinone with a peracid in an inert solvent (Bayer-Villeger reaction) to form 3-(1-hydroxyethyl)-4-acetoxy-2-azetidinone.

3-(1-hydroxyethyl)-4-acetoxy-2-azetidinone is a key intermediate in the preparation of Thienamycin and other carbapenem compounds and is converted into said carbapenem compounds in known manner.

The hydroxy, ethynyl and ring imine groups can be protected during the reaction steps with trialkylsilyl groups.

Shibasaski et al (J.C.S., Chem. Commun. 22 (1982) 1324-1325) disclose the chiral synthesis of (–)-3-[1-(tert.butyldimethylsilyloxy)ethyl]-4-(1-propyn-3-yl)-2-azetidinone using the asymmetric introduction of a phenylthio group to 4-phenylsulphonyl-1-azetidinone as a key step. In particular, said 3-(1-trialkylsilyloxyethyl)-4-propynyl-2-azetidinone is obtained by the following steps :

(a) reaction of 4-acetoxy-2-azetidinone with thiophenol to form (4)-4-phenylthio-2-azetidinone ;

(b) protection of the ring imine group of said (4)-4-phenylthio-2-azetidinone to form (–)-N-(tert.-butyldimethylsilyl)-4-phenylthio-2-azetidinone ;

(c) Aldol condensation of said N-protected-4-phenylthio-2-azetidinone to form diastereomeric N-(tert.butyldimethylsilyl)-3-(1-hydroxyethyl)-4-phenylthio-2-azetidinone ;

(d) Oxidation of said N-protected-3-(1-hydroxyethyl)-4-phenylthio-2-azetidione to form (–)-N-(tert.-butyldimethylsilyl)-3-acetoxy-4-phenylthio-2-azetidinone to form (–)-N-(tert.butyldimethylsilyl)-3-acetoxy-4-phenylthio-2-azetidinone ;

(e) Reduction of said N-protected-3-acetoxy-4-phenylthio-2-azetidinone to form diastereomeric N-(tert.butyldimethylsilyl)-3-(1-hydroxy-ethyl)-4-phenylthio-2-azetidinone and separation of (–) isomer thereof ;

(f) Deprotection of said (–) N-protected-3-(1-hydroxyethyl)-4-phenylthio-2-azetidinone to form (+)-3-(1-hydroxyethyl)-4-phenylthio-2-azetidinone ;

g) Protection of hydroxy group of said 3-(1-hydroxyethyl)-4-phenylthio-2-azetidione by forming

tert.butyldimethylsilyl ether therefrom ;

(h) Oxidation of said 3-(1-tert.butyldimethylsilyloxy)-4-phenylthio-2-azetidinone to form (–)-3-(1-tert.butyldimethylsilyloxy)-4-phenylsulphonyl-2-azetidinone ;

(i) Gridnard reaction of said 3-(1-tert.butyldimethylsilyloxy)-4-phenylsulphonyl-2-azetidinone to form (–)-3[1-(tert.butyldimethylsilyloxy)ethyl]-4-(1-propyn-3-yl)-2-azetidinone.

These methods suffer from the defects that an increased number of steps are required ; that the starting compounds are not easy to produce ; and that the yields are poor.

The present inventors found that the β-lactam compounds having a butynyl group at the 4-position which have not been described in the literature are employable as an important intermediate for 1β-methylcarbapenem compounds and discovered simple and convenient methods for producing the above-described β-lactam compounds, which lead to the establishment of this invention.

This invention is directed to :

(a) a β-lactam compound of the following general formula :

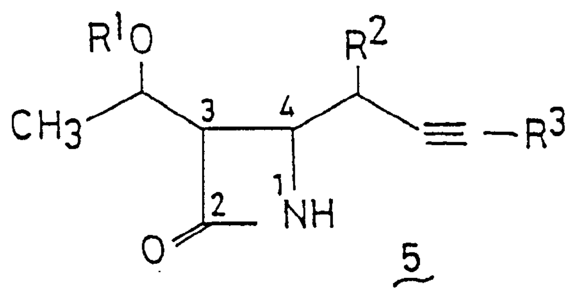

5

wherein $OR^1$ is a hydroxyl group or $R^1$ represents a tri-substituted silyl group of the formula :

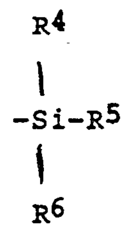

or a carboxylic acid ester group removable by reduction ; $R^2$ is a $C_1$ to $C_7$ alkyl or phenyl group ; $R^3$ is hydrogen or a tri-substituted silyl group of the formula :

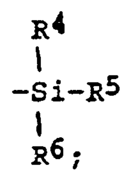

and $R^4$, $R^5$ and $R^6$ each independently represent a $C_1$ to $C_4$ alkyl group or phenyl group, and

(b) a process for producing a β-lactam compound of the following formula :

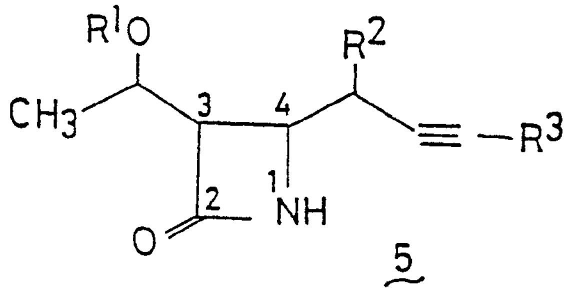

5

wherein $OR^1$, $R^2$ and $R^3$ are as defined hereinbefore which comprises reacting an azetidinone compound represented by the following general formula :

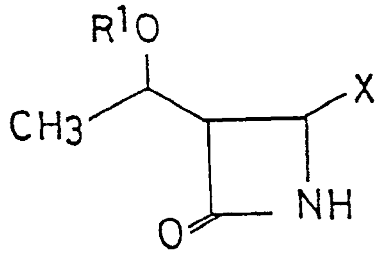

(6)

wherein $OR^1$ is as defined hereinbefore and X is an electronegative group removable through reaction, with a stannylallene represented by the general formula $R^2CH{=}C{=}CH\text{-}SnR^7_3$ (wherein $R^2$ is as defined hereinbefore; $R^7$ is a $C_1$ to $C_4$ alkyl or phenyl group) in the presence of a Lewis acid to give a β-lactam compound of the following general formula :

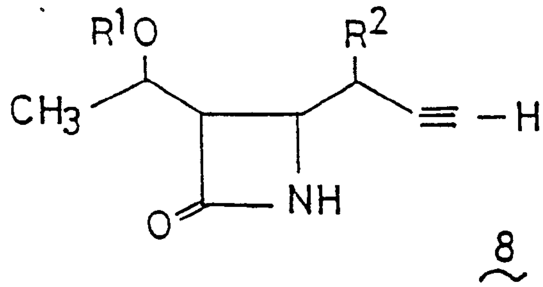

8

wherein $OR^1$ and $R^2$ are as defined hereinbefore ; isomerizing said β-lactam compound by rection with a base and an organosilyl halide represented by the general formula :

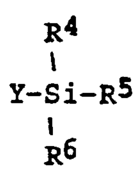

(wherein $R^4$ $R^5$ and $R^6$ are as defined hereinbefore and Y is a halogen), successively, to produce a β-lactam compound of the following general formula :

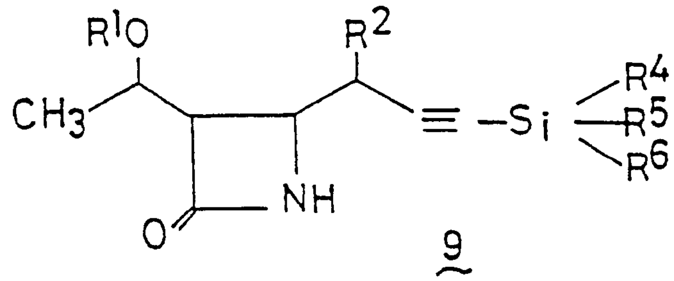

9

wherein $OR^1$, $R^2$, $R^4$, $R^5$ and $R^6$ are as defined hereinbefore, optionally followed by subjecting said B-lactam compound (9) to a removal reaction of the tri-substituted silyl group.

Examples of the protective group represented by $R^1$ include the tri-substituted silyl groups represented by $R^3$, especially tert-butyl-dimethylsilyl, tert-butyldiphenylsilyl and triisopropylsilyl groups, and carboxylic acid ester groups, such as p-nitrobenzyloxycarbonyl and p-methoxybenzyloxycarbonyl groups.

As the $C_1$ to $C_7$ alkyl group represented by $R^2$, there may be mentioned, for example, methyl, ethyl and heptyl groups.

Desirable specific examples of $R^3$ include trimethylsilyl, triethylsilyl, triisopropylsilyl, tert-butyldimethylsilyl and dimethylphenylsilyl groups.

As the $C_1$ to $C_4$ alkyl group being represented individually by $R^4$, $R^5$ and $R^6$, there may be mentioned, for example, methyl, ethyl, propyl, isopropyl, butyl and tert-butyl groups.

Examples of the $C_1$ to $C_4$ alkyl group represented by $R^7$ include methyl, ethyl, propyl and butyl groups.

The β-lactam compounds (5) of this invention are novel and can offer the advantages that the compounds have at the 4-position a butynyl group being convertible into various functional groups and also that the compounds can be produced in the form of β-lactam compounds possessing the desired configuration, for example, compounds (5′) having the same configuration that the one of four consecutive asymmetric carbons possessed by the above-described 1β-methylcarbapenem compounds, when azetidinone compounds having the corresponding configuration are used as a starting compound in the production process according to this invention.

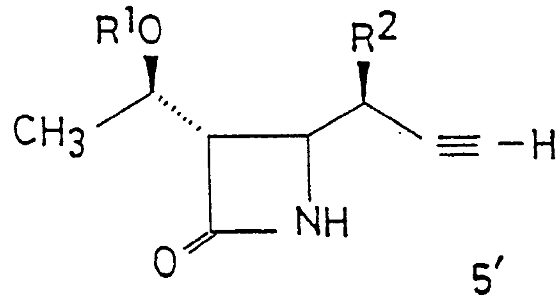

5′

The compounds (8) and (9) of this invention can be produced by the following procedure :

The azetidinone compound (6) is reacted with stannylallene (7) to produce the β-lactam compound (8).

This reaction is carried out in a halogenated hydrocarbon solvent, such as methylene chloride, chloroform and 1,2-dichloroethane, and a hydrocarbon solvent, such as n-hexane, benzene and toluene. Preferable is a halogenated hydrocarbon solvent.

The reaction is allowed to proceed in the presence of a Lewis acid. Preferred Lewis acids are boron trifluoride ethyl etherate [$BF_3 \cdot (C_2H_5)_2O$], trimethylsilyl trifluoromethanesulfonate [$(CH_3)_3 \cdot Si\text{-}OSO_2CF_3$] and tert-butyldimethylsilyl trifluoromethanesulfonate (+>Si-$OSO_2CF_3$).

Usually, the reaction temperature is preferably in the range of −10°C to 60°C.

In this reaction step, for example, when the optically active azetidinone compound (6′) is used as a starting compound to undergo the reaction in the presence of $BF_3 \cdot (C_2H_5)_2O$ or $(CH_3)_3Si\text{-}OSO_2CF_3$ being utilized as a catalyst and in methylene chloride at room temperature, the β-lactam compound (8′) can be obtained almost quantitatively in the following ratios of the α- and β-forms in relation to the configuration of its methyl group :

In the case of $BF_3 \cdot (C_2H_5)_2O$ being used as a catalyst ;

$$\alpha : \beta = 4 : 1$$

In the case of $(CH_3)_3Si\text{-}OSO_2CF_3$ being utilized as a catalyst ;

$\alpha : \beta = 1 : 1$

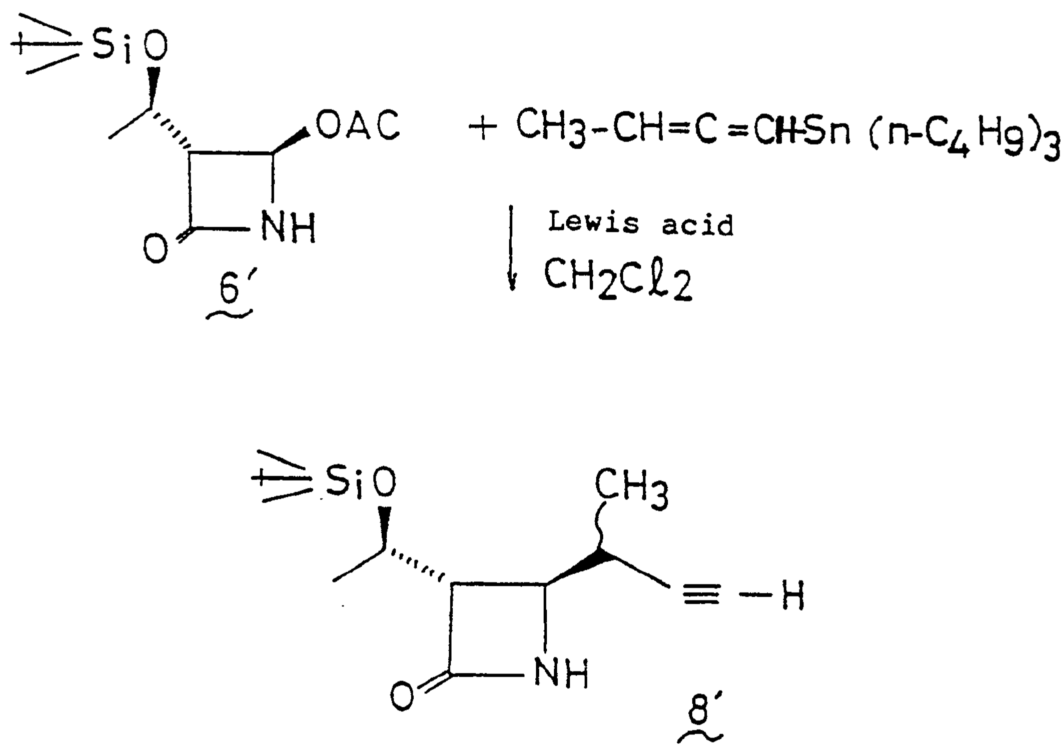


6′

8′

The compound (8) can also be obtained by allowing a metal compound represented by the formula

$$R^2-\overset{M}{C}H\ C\equiv CH$$

(wherein $R^2$ is as defined hereinbefore ; M is —Mg · X (X is halogen), Li, or a titanic acid ester or boric acid ester group, in place of stannylallene, to act on the compound (6). This reaction proceeds without addition of Lewis acid.

The β-lactam compound (8) as produced in the preceding step is reacted with a base and organosilyl halide, successively.

As the base, there are preferably employed lithium alkylamides, such as lithium diisopropylamide, and alkyl lithium, such as n-butyllithium-tetramethylethylenediamine, sec-butyllithium and tert-butyllithium.

The reaction is carried out in an anhydrous, inactive solvent, such as anhydrous tetrahydrofuran.

The resulting reaction product is subsequently reacted with an organosilyl halide represented by the formula

$$Y-\underset{R^6}{\overset{R^4}{Si}}-R^5$$

(wherein $R^4$, $R^5$ and $R^6$ are the same or different and each represents a $C_1$ to $C_4$ alkyl or phenyl group ; Y is a halogen) to produce the β-lactam compound (9).

Examples of the organosilyl halide include trimethylchlorosilane, triethylchlorosilane, tripropylchlorosilane, tert-butyldimethylchlorosilane and triphenylchlorosilane.

In the course of this reaction, the α-form of the compound (8) wherein the alkyl or phenyl group represented by $R^2$ is involved in the configuration isomerizes to the β-form.

When the above-described compound (8′) having the α-form/β-form ratio of 1 : 1, being used as a starting compound in this reaction step, is reacted with 3.5 equivalents each of lithium diisopropylamide and

trimethylchlorosilane successively in anhydrous tetrahydrofuran at −78°C, for example, the α-form isomerizes to the β-form, resulting the high yield of the compound (9′) containing the α-form : β-form (1 : 13).

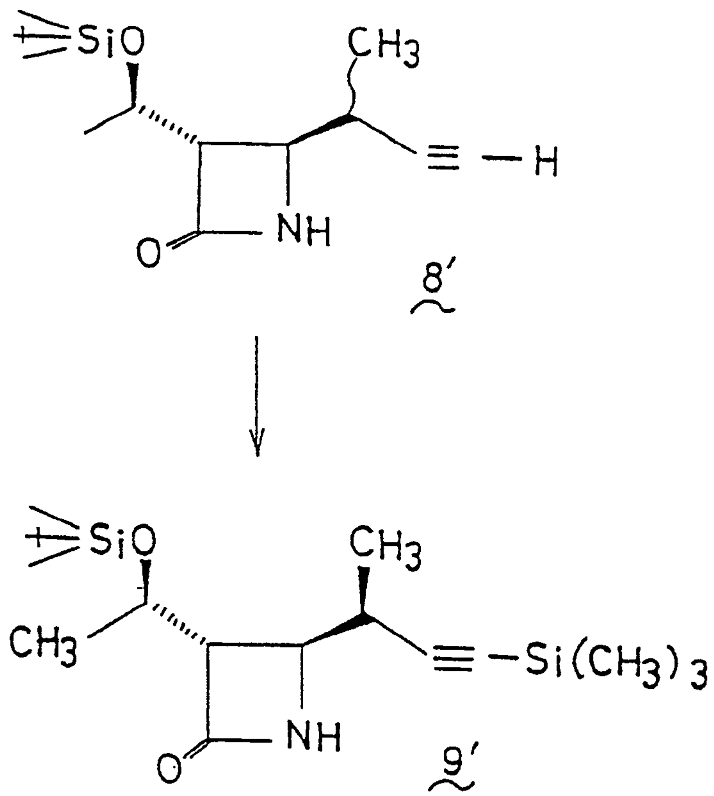

8′

9′

The compound (9) obtained thus can be derived to the compound (5) through a removal reaction for the trisubstituted silyl group.

This reaction can be carried out in easy and quantitative manner by allowing silver nitrate and potassium cyanate successively to act on the compound (9) in a water-containing solvent or alternatively by allowing potassium fluoride to act on the compound in aqueous methanol.

By the above described procedure, for example, the compound (9′) mentioned hereinbefore can be converted into the compound (8″).

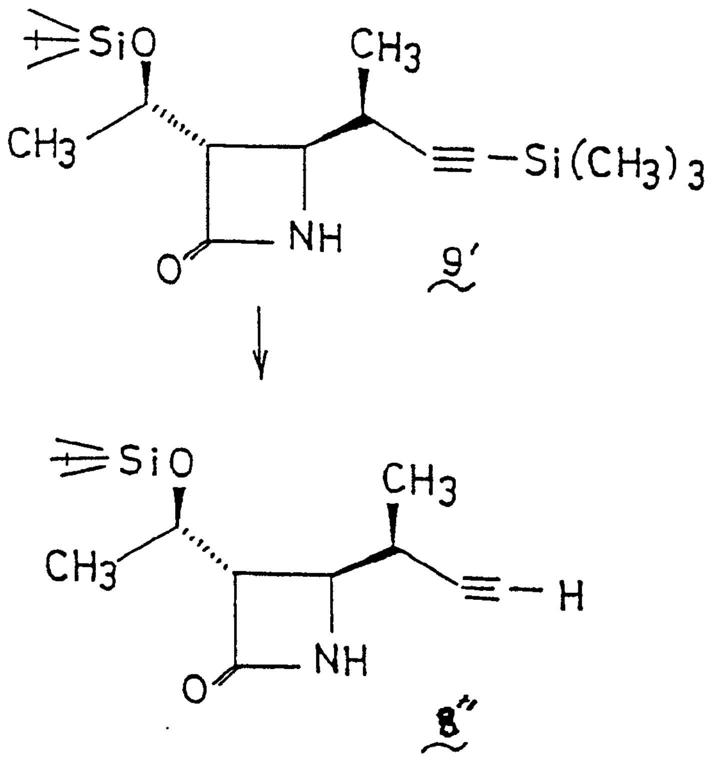

9'

8''

In the same manner as described above, the compound having a phenyl group instead of the methyl group in the substituent at 4-position of the azetidinone ring of the compound (8'') can be obtained by employing 3-phenyl-1-tributylstannylallene in place of 3-methyl-1-tributyl-stannylallene.

The compounds of this invention can be derived to the important intermediate (4) as described hereinbefore, for example, through the reaction steps being illustrated below by the reaction formulae :

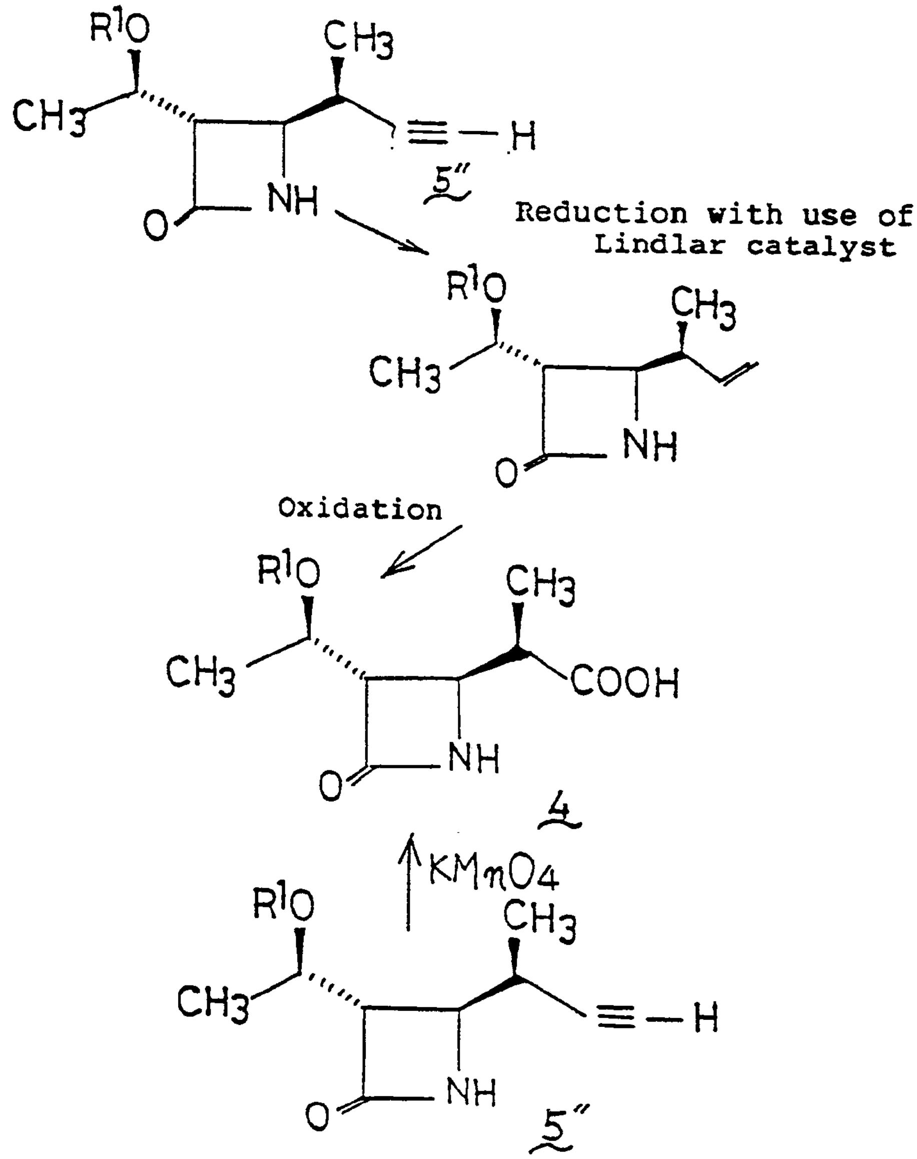


In addition, the compounds of this invention can be converted to optically active azetidinones being derivable into 1β-methylcarbapenem compounds other than those as described above.

The β-lactam compounds (5) having a butynyl group at the 4-position as obtained according to this invention can not only be easily derived to the azetidinone compounds (4), important intermediates for synthesizing for example 1β-methylcarbapenem (3), but also be produced in shortened steps in improved yields by starting from azetidinone compounds which are easily available or producible.

Below described are examples and reference examples to illustrate more specifically this invention.

Reference Example 1

3-Methyl-1-tributylstannylallene ($CH_3$-CH=C=CHSnBu$_3$)

1-Bromo-3-methylallene (9.69 g) is added to 100 ml of anhydrous ether under a stream of nitrogen, followed by cooling to −78°C. n-Butyllithium (10 w/w% n-hexane solution) (46.7 ml) is added to the mixture under stirring over the period of 15 minutes, followed by stirring at the same temperature for 1 hour. After adding anhydrous tetrahydrofuran (20 ml), chlorotributylstannane (23.7 g) is added to the mixture over a period of 10 minutes, and the reaction mixture is allowed to warm up spontaneously to room temperature, followed by stirring at room temperature for 15 minutes. The reaction solution is poured into water, and extraction is performed with ether. The organic layer is washed with water and saturated aqueous sodium hydrochloride solution, successively,

and dried over anhydrous magnesium sulfate. The solvent is removed under reduced pressure, and the residue is purified through silica gel column chromatography (n-hexane) to give 17.4 g of the objective compound.

IR ($CHCl_3$) : 1930 $cm^{-1}$

NMR ($CDCl_3$) δ : 0.7 to 2.0 (27 H,m), 1.65 (3H, dd, J = 4,7 Hz), 4.23 (1H, dq, J = 7,7 Hz), 5.05 (1H, dq, J = 4,7 Hz)

Example 1

(3S, 4R)-4-(1-Butyn-3-yl)-3-[(1R)-1-(tert-butyldimetylsilyloxy)ethyl]-2-azetidinone (5''')

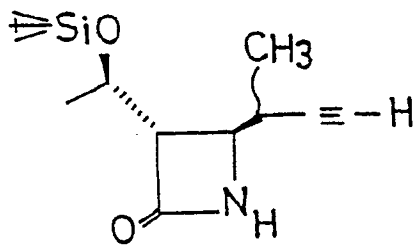

α : β = 1 : 1

5'''

(3R, 4R)-4-Acetoxy-3-[(1R)-1-(tert-butyldimethylsilyloxy)ethyl]-2 azetidinone (653 mg) is added to 20 ml of anhydrous methylene chloride under a stream of nitrogen, followed by cooling with ice. After adding 3-methyl-1-tributylstannylallene (1.56 g), trimethylsilyl trifluoromethanesulfonate (0.35 ml) is added to the mixture, followed by stirring at the same temperature for 15 minutes and then at room temperature for 14 hours. The reaction solution is concentrated under reduced pressure, and the concentrate is admixed with ether. The mixture is washed with phosphoric acid buffer (pH 7) and admixed with saturated aqueous potassium fluoride solution, followed by stirring vigorously for 1 hour.

The resulting precipitate is filtered out, and the filtrate is dried over anhydrous magnesium sulfate, followed by removal of the solvent under reduced pressure. The residue is purified through silica gel column chromatography (n-hexane : ether = 1 : 1) to give 626 mg of a 1 : 1 mixture of (3S, 4R)-4-[1-butyn-3(R)-yl]-3-[(1R)-1-(tert-butyldimethylsilyloxy)ethyl]-2-azetidinone (α-methyl form) and (3S,4R)-4-[1-butyn-3(S)-yl]-3-[(1R)-1-(tert-butyldimethylsilyloxy)ethyl]-2-azetidinone (β-methyl form).

IR ($CHCl_3$) : 3420, 3300, 1755 $cm^{-1}$

NMR (90 MHz) ($CDCl_3$) δ : 0.08 (6 H, S), 0.88 (9 H, S), 1.24 (6 H, d, J = 6 Hz), 2.12 (1 H, d, J = 2.5 Hz), 2.59 (1/2 H, m, α-isomer), 2.70 (1/2 H, m, β-isomer), 2.84 (1/2, m, α-isomer), 3.00 (1/2 H, m, β-isomer), 3.57 (1/2 H, dd, J = 7.6, 1.8 Hz, α-isomer), 3.67 (1/2 H, dd, J = 6.4, 2.0 Hz, β-isomer), 4.16-4.26 (1 H, m)

Exact mass spectrometry : as $C_{15}H_{27}NO_2Si-C_4H_9$

| | |
|---|---|
| Calcd. : | 224.1108 |
| Found : | 224.1108 |

Example 2

By following the same procedure as described in Example 1, 0.44 ml of boron trifluoride ethyl etherate (47% ether solution) is used in place of trimethylsilyl fluoromethanesulfonate to produce the compound (5''') (α-methyl form : β-methyl form = 4 : 1). Yield of 569 mg.

Example 3

(3S, 4R)-4-[1-Butyn-3(S)-yl]-3-[(1R)-1-(tert-butyl-dimethylsilyloxy)ethyl]-2-azetidinone (5'')

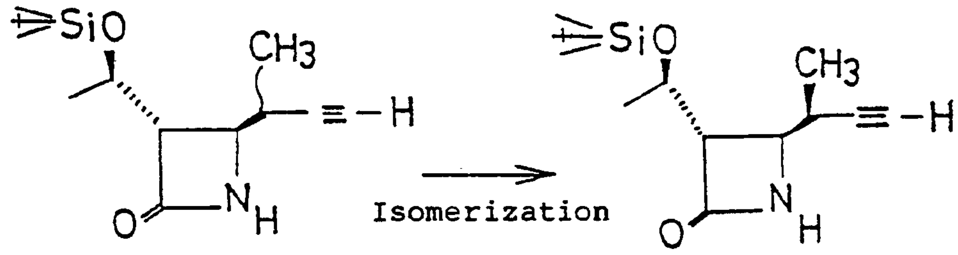


α : β = 1 : 1

5''' 5''''

Diisopropylamine (0.34 ml) is added to anhydrous tetrahydrofuran (4.4 ml) under a stream of nitrogen, followed by cooling with ice. n-Butyllitithium (10 w/w% n-hexane solution) (1.6 ml) is added to the solution, followed by stirring at the same temperature for 30 minutes. The solution is cooled to −78°C and admixed with a solution in anhydrous tetrahydrofuran (4.4 ml) of the mixture (5'') (196 mg) of the α-methyl form and β-methyl form as obtained in Example 1, and the mixed solution is warmed up to −30°C over a 1 hour period, then cooled down to −78°C again and admixed with trimethylchlorosilane (0.31 ml). The reaction solution is allowed to warm up to room temperature spontaneously, and admixed with water and ethyl acetate, and the water layer separated is extracted with ethyl acetate. The combined organic layer is washed with water and saturated aqueous sodium chloride solution, successively and dried over anhydrous magnesium sulfate, followed by removal of the solvent under reduced pressure. The resulting residue is purified through silica gel column chromatography (n-hexane: ethyl acetate = 4 : 1) to give 164 mg of (3S, 4R)-4-[trimethylsilyl-1-butyn-3-(S)-yl]-3-[(1R)-1-(tert-butyldimethylsilyloxy)ethyl]-2-azetidinone.

IR ($CHCl_3$) : 3425, 2160, 1760

NMR (200 MHz) ($CDCl_3$) δ : 0.07 (3 H, S), 0.08 (3 H, S), 0.13 (9 H, S), 0.88 (9 H, S), 1.23 (6 H, t, J = 7 Hz), 2.66 (1 H, quint, J = 7 Hz), 2.97 (1 H, bs), 3.63 (1 H, dd, J = 2,7 Hz), 4.20 to 4.27 (1 H, m), 6.37 (1 H, bs).

Exact mass spectrometry : as $C_{18}H_{35}NO_2Si_2$-$C_4H_9$

| | |
|---|---|
| Calcd. : | 296.1499 |
| Found : | 296.1492 |

The substance is dissolved in ethanol (1.4 ml), and the solution is cooled with ice and admixed gradually with a solution of silver nitrate (178 mg) in ethanol-water (1 : 3) (1.7 ml). After stirring for 15 minutes, 9M aqueous potassium cyanide solution (0.58 ml) is added to the solution, followed by stirring for 10 minutes and then at room temperature for 1 hour.

The reaction solution is admixed with ether (11 ml) and water (11 ml), and the aqueous layer is extracted with ether. The organic layer is washed with water and dried over anhydrous magnesium sulfate, and the solvent is removed under reduced pressure, followed by purification of the residue through silica gel column chromatography (n-hexane : ether = 2 : 1) to give 127 mg of the desired compound.

IR ($CHCl_3$) : 3420, 3300, 1755 $cm^{-1}$

NMR (200 MHz) ($CDCl_3$) δ : 0.07 (3 H, S), 0.08 (3 H, S), 0.88 (9 H, S), 1.24 (3 H, d, J = 6.8 Hz), 1.25 (3 H, d, J = 6.2 Hz), 2.12 (1 H, d, J = 2.4 Hz), 2.70 (1 H, d, of quint, J = 2.4, 6.8 Hz), 2.99 to 3.02 (1 H, m), 3.67 (1H, dd, J = 2, 6.2 Hz), 4.23(1 H, dq, J = 6.2, 4 Hz), 6.00 (1 H, brs).

Exact mass spectrometry : as $C_{15}H_{27}NO_2Si$-$C_4H_9$

| | |
|---|---|
| Calcd. : | 224.1108 |
| Found : | 224.1108 |

Reference Example 2

3-Phenyl-1-tributylstannylallene

1-Phenyl-2-propyn-1-ol (1.32 g) is converted to mesylate in accordance with the conventional method while using methanesulfonyl chloride and triethylamine. Tributylstannyllithium as produced by acting on bis-tributylstannane (5.8 g) and an equimolar amount of n-butyllithium (10 w/v% n-hexane solution) (6.4 ml) in tetrahydrofuran (10 ml) is added to a solution of cuprous bromide (1.43 g) and lithium bromide (0.87 g) in anhydrous tetrahydrofuran (30 ml) at −60°C to prepare an organic tin reagent (n-$Bu_3SnLi$), to which a solution of the above-described mesylate in anhydrous tetrahydrofuran (5 ml) is added at −60°C, followed by stirring for 2 hours. The reaction solution is poured into saturated aqueous ammonium chloride solution, and extraction is performed with ether. The ether layer is dried over anhydrous magnesium sulfate, and the solvent is removed under reduced pressure. The resulting residue is purified by column chromatography on 6% aqueous alumina (n-hexane) to give 2.8 g of the objective compound.

IR ($CHCl_3$) : 1920, 1595 $cm^{-1}$

NMR (90 MHz) ($CDCl_3$) δ : 0.7 to 1.8 (27 H, m), 5.64 (1 H, d, J = 7 Hz), 5.82 (1H, d, J = 7 Hz), 7.32 to 7.56 (5 H, m).

Example 4

(3S, 4R)-4-(3-Phenyl-1-propyn-3-yl)-3-[(1R)-1-(tert-butyldimethylsilyloxy)ethyl]-2-azetidinone

By following the same procedure as described in Example 2 except that 3-phenyl-1-tributylstannylallene (1.84 g) is used in place of 3-methyl-1-tributylstannylallene, there is obtained the objective compound. Yield of 640 mg.

IR ($CHCl_3$) : 3425, 3320, 1765 $cm^{-1}$

NMR (500 MHz) ($CDCl_3$) δ : 0.04 (6 H, S), 0.75 (12/7 H, d, J = 6.7 Hz), 0.86 (9 H, S), 1.04 (16/7 H, d, J = 6.7 Hz), 2.35(1 H, t, J = 2.4 Hz, 2.99 to 3.03 (1 H, m), 3.77 (4/7 H, t, J = 1.8 Hz), 3.78 (3/7 H, t, J = 2.4 Hz), 3.84 (4/7 H, dd, J = 6.7, 2.2 Hz), 3.90 (3/7 H, dd, J = 7.9, 2.2 Hz), 4.11 (4/7 H, dq, J = 4.3, 6.7 Hz), 4.22 (3/7 H, dq, J = 3.1, 6.7 Hz), 7.34 to 7.41 (5 H, m)

Example 5

(3S, 4R) -4-(3-Phenyl-1-trimethylsilyl-1-propyn-3 (R)-yl]-3-[(1R) -1-(tert-butyldimethylsilyloxy) ethyl]-2-azetidinone

By following the first half of the same procedure as described in Example 3 except that 240 mg of the product as obtained in Example 4 is employed, with lithium diisopropylamide being used as a base, the isomerization reaction through silylation is carried out to produce 102 mg of the objective compound.

IR ($CHCl_3$) : 3410, 2180, 1760 $cm^{-1}$

NMR (90 MHz) ($CDCl_3$) δ : 0.04 (6 H,S), 0.13 (9 H, S), 0.88 (3 H, d, J = 6 Hz), 0.91 (9 H, S), 3.00 to 3.10 (1 H, m), 3.80 to 3.90 (2 H, m), 4.10 to 4.34 (1 H, m), 7.36 (5 H, S).

Example 6

(3S, 4R)-4-(3-Phenyl-1-propyn-3(R)-yl]-3-[(1R)-1-(tert-butyldimethylsilyloxy)ethyl]-2-azetidinone

By following the last half of the same procedure as described in Example 3, 100 mg of the product as obtained in Example 5 is subjected to a removal reaction for silylgroup to give 44 mg of the objective compound.

IR ($CHCl_3$) : 3425, 8320, 1765 $cm^{-1}$

NMR (500 MHz) ($CDCl_3$) δ : 0.04 (6 H, S), 0.75 (3 H, d, J = 6.7 Hz), 0.86 (9 H, S), 2.35 (1 H, d, J = 2.4 Hz), 2.99 to 3.03 (1 H, m), 3.78 (1 H, t, J = 2.4 Hz), 3.90 (1 H, dd, J = 7.9, 2.2 Hz), 4.22(1 H, dq, J = 3.1, 6.7 Hz), 7.34 to 7.40 (5 H, m).

Example 7

(3S, 4R)-4-[1-Buten-3(S)-yl]-3-[(1R)-1-(tert-butyldimethylsilyloxy)ethyl]-2-azetidinone

The β-lactam compound (5'''') (81 mg) as produced in in Example 3 is dissolved in methanol (1 ml), and 5% Pd-$BaSO_4$ (1.6 mg) and quinoline (1.6 mg) are added to die solution, which is then allowed to absorb about 6.5 ml of hydrogen under atmospheric pressure at ambient temperature. The reaction solution is filtered, and the filtrate is admixed with ether and water to extract the aqueous layer with ether. The organic layer is washed with water and dried over anhydrous magnesium sulfate.

The solvent is removed under reduced pressure, and the residue is purified through silica gel column chromatography (n-hexane : ether = 2 : 1) to give 73.4 mg of the objective compound.

IR ($CHCl_3$) : 3410, 1750 $cm^{-1}$

NMR (200 MHz) ($CDCl_3$) δ : 0.07 (6 H, S), 0.88 (9 H, S), 1.07 (3 H, d, J = 7 Hz) · 1.17 (3 H, d, J = 6 Hz), 2.33 (1 H, brq, J = 7 Hz), 2.82 (1 H, ddd, J = 4.5, 2.5, 1 Hz), 3.52 (1 H, dd, J = 8, 2.5 Hz) 4.18 (1 H, dq, J = 6, 4.5 Hz), 4.96 to 5.22 (2 H, m), 5.60 to 6.00 (2 H, m).

Exact mass spectrometry : as $C_{11}H_{20}NO_2Si$-$C_4H_9$

| | |
|---|---|
| Calcd. : | 226.126 |
| Found : | 226.128 |

## Claims

1. A β-lactam compound of the following general formula :

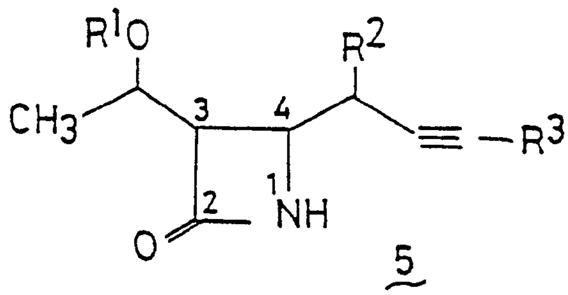

5

wherein $OR^1$ is a hydroxyl group or $R^1$ represents a tri-substituted silyl group of the formula :

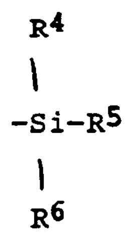

or a carboxylic acid ester group removable by reduction ; $R^2$ is a $C_1$ to $C_7$ alkyl or phenyl group ; $R^3$ is hydrogen or a tri-substituted silyl group of the formula :

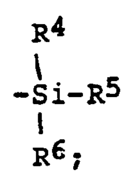

and $R^4$, $R^5$ and $R^6$ each independendy represent a $C_1$ to $C_4$ alkyl group or phenyl group.

2. A compound as claimed in Claim 1, wherein $R^1$ is a said tri-substituted silyl group or a carboxylic acid

ester group removable by reduction and $R^3$ is a said tri-substituted silyl group.

3. A compound as claimed in Claim 1, wherein $R^1$ is a said tri-substituted silyl group ; $R^2$ is methyl ; and $R^3$ is hydrogen.

4. A compound as claimed in Claim 1, wherein $R^1$ is tert-butyldimethylsilyl and $R^3$ is hydrogen.

5. A compound as claimed in any one of the preceding claims, wherein said compound has a configuration represented by the following formula :

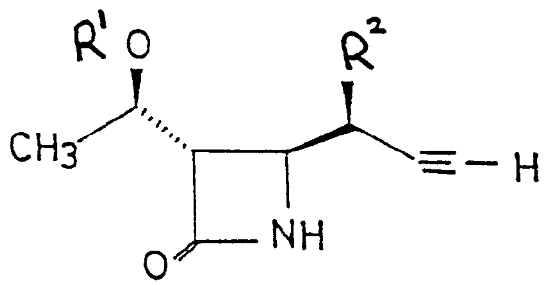

wherein $OR^1$, $R^2$ and $R^3$ are as defined in the relevant preceding claim.

6. A process for producing a β-lactam compound of the following formula :

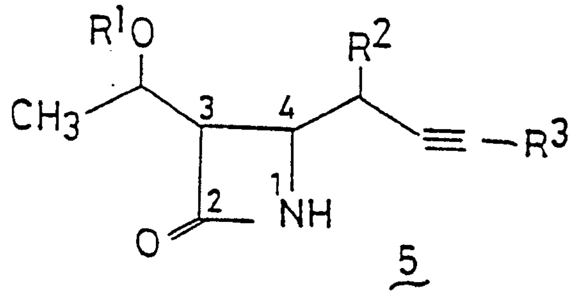

5

wherein $OR^1$, $R^2$ and $R^3$ are as defined in Claim 1, which comprises reacting an azetidinone compound represented by the following general formula :

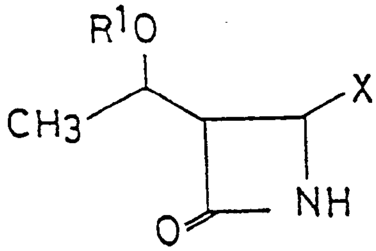

(6)

wherein $OR^1$ is as defined hereinbefore and X is an electronegative group removable through reaction, with a stannylallene represented by the general formula $R^2CH{=}C{=}CH\text{-}SnR^7_3$ (wherein $R^2$ is as defined hereinbefore; $R^7$ is a $C_1$ to $C_4$ alkyl or phenyl group) in the presence of a Lewis acid to give a β-lactam compound of the following general formula :

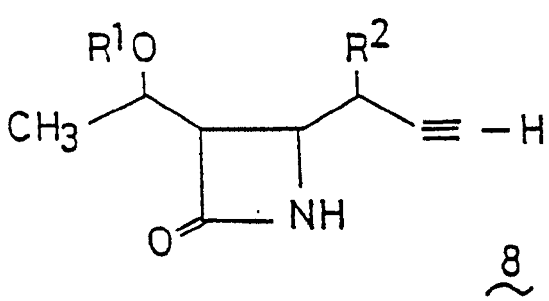

8

wherein $OR^1$ and $R^2$ are as defined hereinbefore ; isomerizing said β-lactam compound by reaction with a base and an organosilyl halide represented by the general formula :

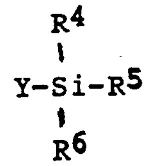

(wherein $R^4$, $R^5$ and $R^6$ are as defined in Claim 1 ; and Y is a halogen), successively, to produce a β-lactam compound of the following general formula :

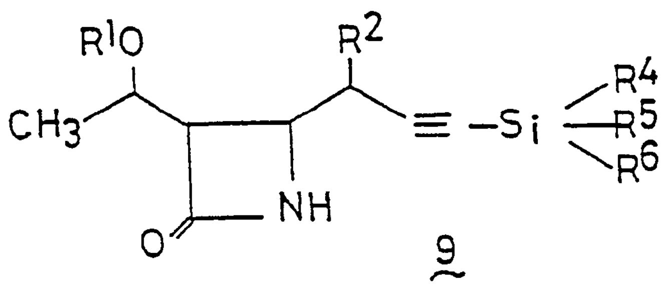

9

wherein $OR^1$, $R^2$, $R^4$, $R^5$ and $R^6$ are as defined hereinbefore, optionally followed by subjecting said β-lactam compound (9) to a removal reaction of the tri-substituted silyl group.

7. A process as claimed in Claim 6, wherein $R^7$ is n-butyl group and X is $CH_3COO$ group.

8. A process as claimed in Claim 6 or Claim 7, wherein the azetidinone compound (6) has a configuration of the following formula :

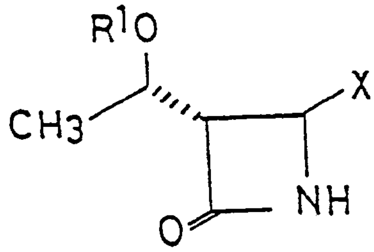

(6)

whereby the β-lactam compound (8) has a configuration of the following formula :

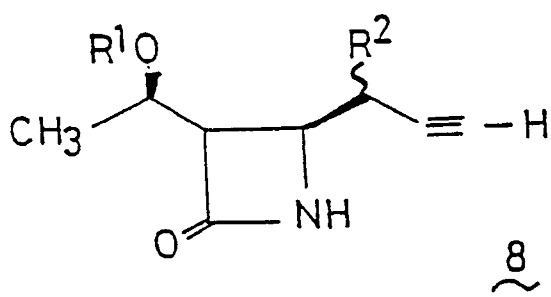

8

the β-lactam compound (9) has a configuration of the following formula :

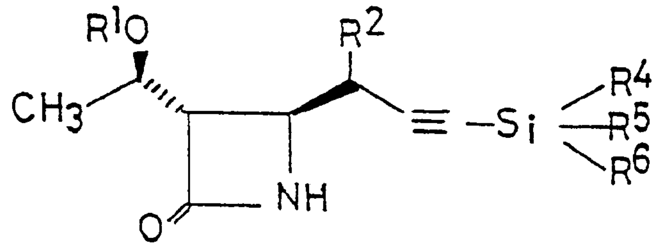

; and

the β-lactam compound resulting from removal of the tri-substituted silyl groups has a configuration of the following formula :

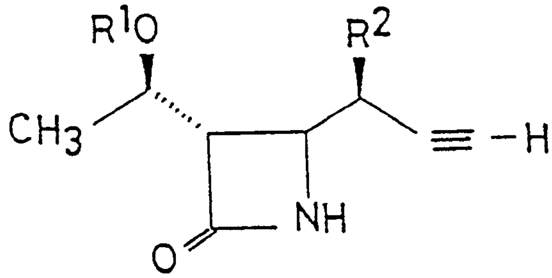

9. A process for producing an azetidinone of the formula :

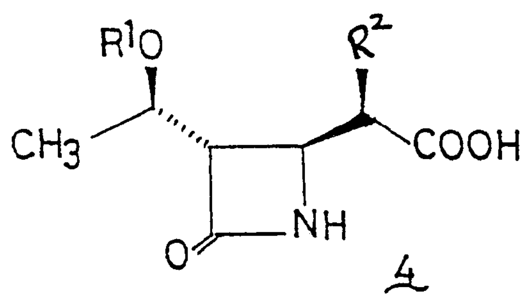

4

(wherein $R^1$ and $R^2$ are as defined in Claim 1) which comprises treating a β-lactam of the formula :

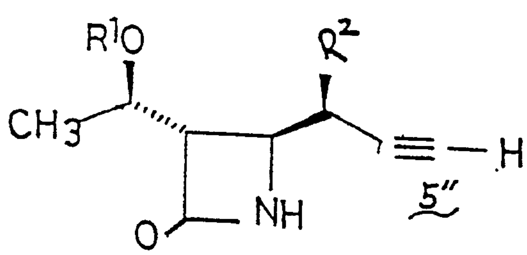

5″

wherein $OR^1$ and $R^2$ are as defined in Claim 1 to convert the ethynyl group into a carboxylic acid group.

**Revendications**

1. Dérivé de β-lactam répondant à la formule générale suivante :

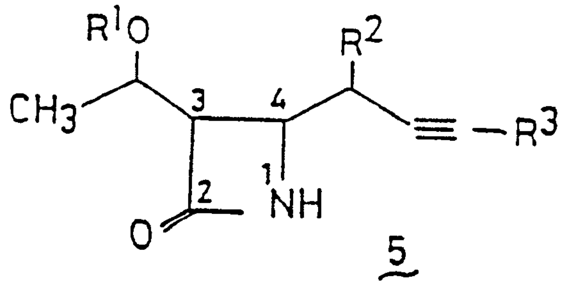

5

dans laquelle $OR^1$ est un groupement hydroxy ou bien $R^1$ représente un groupement silyle trisubstitué de formule

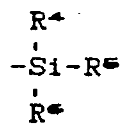

ou un groupement ester d'acide carboxylique éliminable par réduction ; $R^2$ est un groupement alkyle en $C_1$ à $C_7$ ou phényle ; $R^3$ est un atome d'hydrogène ou un groupement silyle trisubstitué de formule

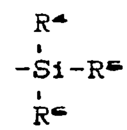

et $R^4$, $R^5$ et $R^6$ représentent chacun, indépendamment les uns des autres, un groupement alkyle en $C_1$ à $C_4$ ou phényle.

2. Composé selon la revendication 1, dans lequel $R^1$ est un groupement silyle trisubstitué du genre défini ou un groupement ester d'acide carboxylique éliminable par réduction et $R^3$ est un groupement silyle trisubstitué du genre défini.

3. Composé selon la revendication 1, dans lequel $R^1$ est un groupement silyle trisubstitué du genre défini, $R^2$ est un groupement méthyle et $R^3$ est un atome d'hydrogène.

4. Composé selon la revendication 1, dans lequel $R^1$ est un groupement tert.-butyldiméthylsilyle et $R^3$ est un atome d'hydrogène.

5. Composé selon l'une quelconque des revendications 1 à 4, ayant une configuration représentée par la formule suivante

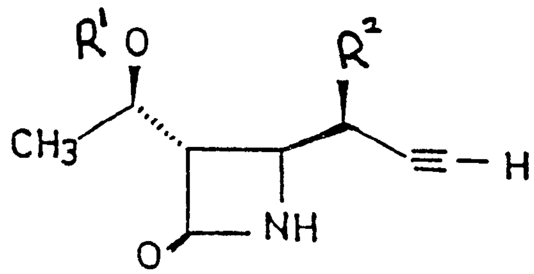

dans laquelle $OR^1$, $R^2$ et $R^3$ sont tels que définis dans la revendication précédente concernée.

6. Procédé de préparation d'un dérivé de β-lactame répondant à la formule suivante

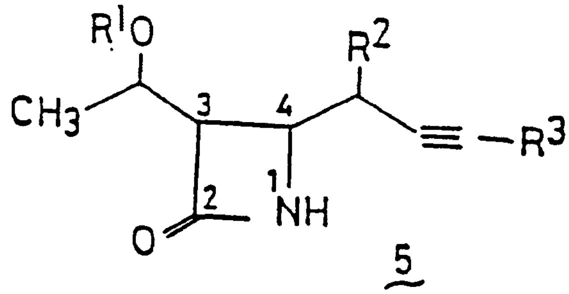

5

dans laquelle $OR^1$, $R^2$ et $R^3$ sont tels que définis dans la revendication 1, comprenant les opérations consistant à faire réagir un dérivé d'azétidinone représenté par la formule genérale suivante

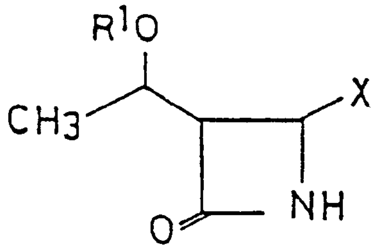

(6)

dans laquelle $OR^1$ est tel que défini précédemment et X est un groupement électronégatif éliminable par réaction, avec un stannylallène représenté par la formule génerale $R^2CH{=}C{=}CH\text{-}SnR^7{}_3$ (dans laquelle $R^2$ est tel que défini précédemment ; $R^7$ est un groupement alkyle en $C_1$ à $C_4$ ou phényles) en présence d'une acide de Lewis, pour donner un dérivé de β-lactame représenté par la formule générale suivante

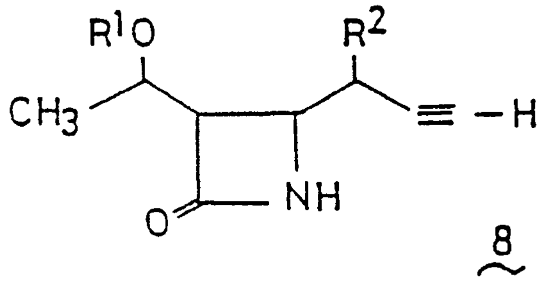

8

dans laquelle $OR^1$ et $R^2$ sont tels que définis précédemment ; à isomériser ledit dérivé de β-lactame par réaction successive avec une base et avec un halogénure d'organo-silyle représenté par la formule générale

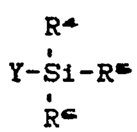

(dans laquelle $R^4$, $R^5$ et $R^6$ sont tels que définis précédemment et Y est un atome d'halogène), pour produire un dérivé de β-lactame représenté par la formule générale suivante

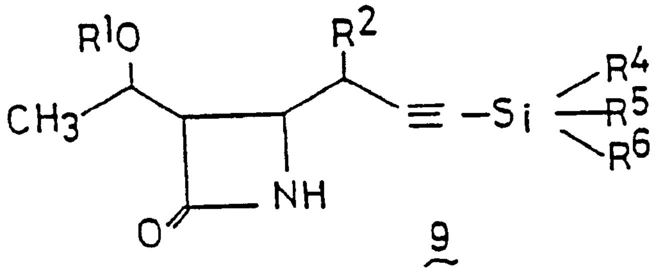

9

dans laquelle $OR^1$, $R^2$, $R^4$, $R^5$ et $R^6$ sont tels que définis précédemment, puis à soumettre éventuellement ledit dérivé de β-lactame (9) à une réaction d'élimination du groupement silyle trisubstitué.

7. Procédé selon la revendication 6, dans lequel $R^7$ est un groupement n-butyle et X est un groupement $CH_3COO$.

8. Procédé selon la revendication 6 ou 7, dans lequel le dérivé d'azétidinone (6) a une configuration représentée par la formule suivante :

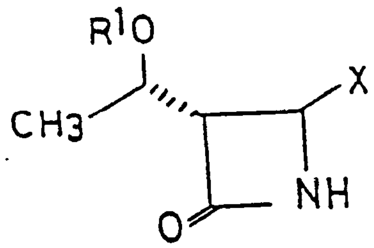

(6)

ce qui fait que le dérivé de β-lactame (8) a une configuration représentée par la formule suivante :

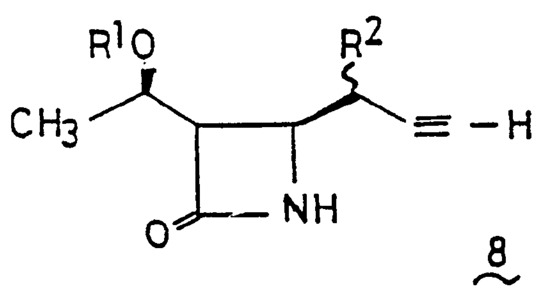

8

que le dérivé de β-lactame (9) a une configuration représentée par la formule suivante :

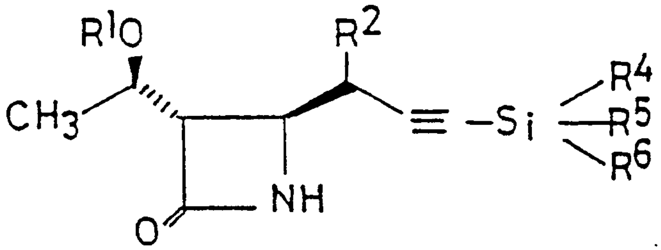

et que le dérivé de β-lactame résultant de l'élimination des groupements silyle trisubstitués a une configuration représentée par la formule suivante :

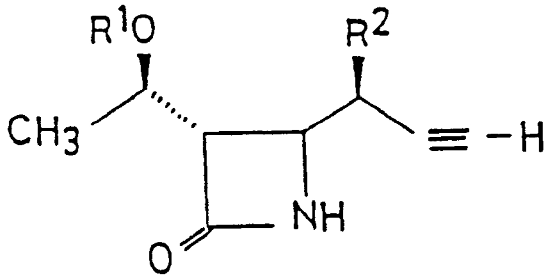

9. Procédé de préparation d'une azétidinone de formule

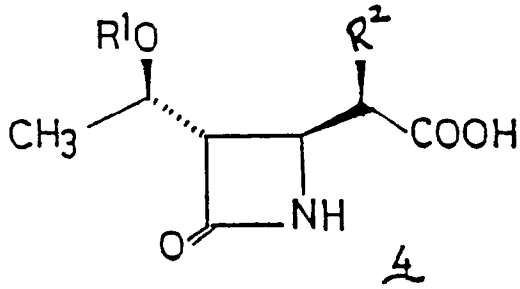

4

(dans laquelle $R^1$ et $R^2$ sont tels que définis dans la revendication 1), comprenant l'opération consistant à traiter un β-lactame de formule

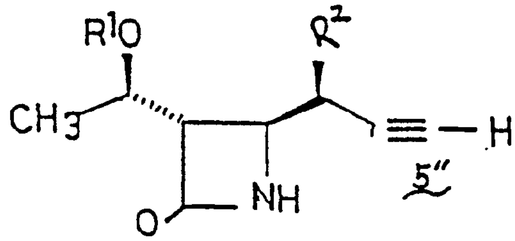

5''

dans laquelle $OR^1$ et $R^2$ sont tels que définis dans la revendication 1, pour convertir le groupement éthynyle en un groupement acide carboxylique.

## Patentansprüche

1. β-Lactam-Verbindung der folgenden allgemeinen Formel :

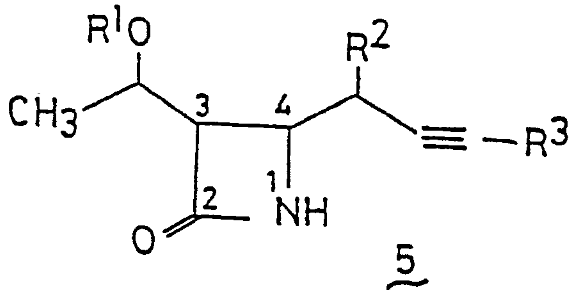

5

in der $OR^1$ eine Hydroxylgruppe ist oder $R^1$ eine trisubstituierte Silylgruppe der Formel :

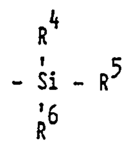

oder eine durch Reduzierung entfernbare Carbonsäureestergruppe bedeutet ; $R^2$ eine $C_1$ bis $C_7$-Alkylgruppe oder Phenylgruppe ist ; $R^3$ Wasserstoff oder eine trisubstituierte Silylgruppe der Formel :

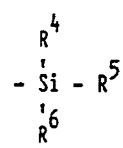

ist und $R^4$, $R^5$ und $R^6$ jeweils unabhängig eine $C_1$ bis $C_4$-Alkylgruppe oder eine Phenylgruppe bedeuten.

2. Verbindung nach Anspruch 1, in der $R^1$ eine trisubstituierte Silylgruppe oder eine durch Reduzierung entfernbare -Carbonsäureestergruppe ist und $R^3$ eine trisubstituierte Silylgruppe ist.

3. Verbindung nach Anspruch 1, in der $R^1$ eine trisubstituierte Silylgruppe ist, $R^2$ Methyl ist und $R^3$ Wasserstoff ist.

4. Verbindung nach Anspruch 1, in der $R^1$ tert.-Butyldimethylsilyl ist und $R^3$ Wasserstoff ist.

5. Verbindung nach einem der vorhergehenden Ansprüche, wobei die Verbindung eine Konfiguration entsprechend der folgenden Formel aufweist :

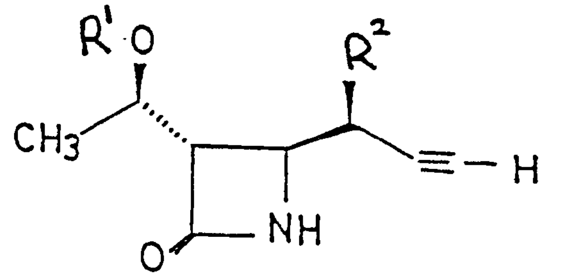

in der $OR^1$, $R^2$ und $R^3$ die in dem entsprechenden vorhergehenden Anspruch gegebene Definition besitzen.

6. Verfahren zur Herstellung einer β-Lactam-Verbindung der folgenden Formel :

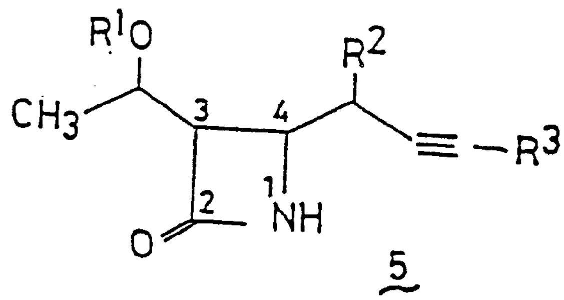

5

in der $OR^1$, $R^2$ und $R^3$ die in Anspruch 1 gegebene Definition aufweisen, bei dem man eine Azetidinon-Verbindung der folgenden allgemeinen Formel :

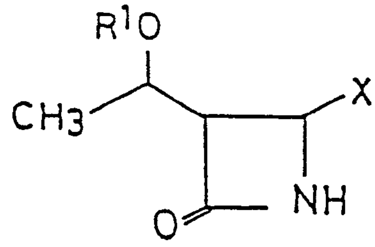

(6)

in der $OR^1$ die vorstehend gegebene Bedeutung besitzt, und X eine durch Umsetzen entfernbare elektronegative Gruppe ist, mit einem Stannylallen der allgemeinen Formel $R^2CH{=}C{=}CH\text{-}SnR^7{}_3$ (in der $R^2$ die vorstehend gegebene Bedeutung aufweist und $R^7$ eine $C_1$ bis $C_4$-Alkylgruppe oder Phenylgruppe ist), in Gegenwart einer Lewis-Säure umsetzt, um eine β-Lactam-Verbindung der folgenden allgemeinen Formel :

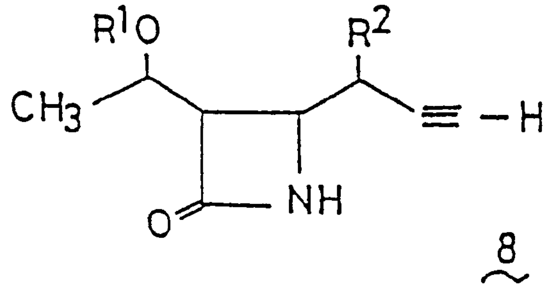

8

in der $OR^1$ und $R^2$ die vorstehend gegebene Bedeutung aufweisen, herzustellen, die β-Lactam-Verbindung durch sukzessives Umsetzen mit einer Base und einem Organosilylhalogenid entsprechend der allgemeinen Formel :

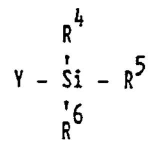

(in der $R^4$, $R^5$ und $R^6$ die in Anspruch 1 gegebene Definition besitzen und Y ein Halogen ist) isomerisiert, um eine β-Lactam-Verbindung der folgenden Formel :

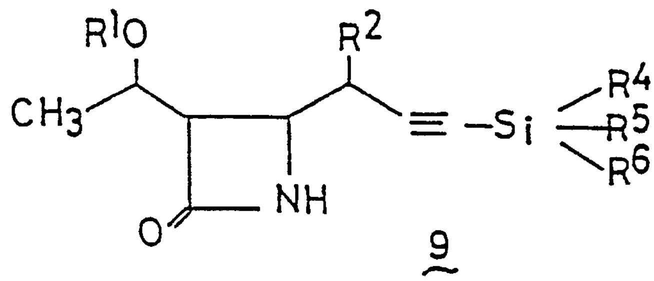

9

in der $OR^1$, $R^2$, $R^4$, $R^5$ und $R^6$ die vorstehend gegebene Bedeutung aufweisen, herzustellen und gegebenenfalls die β-Lactam-Verbindung (9) anschließend einer Reaktion unterwirft, um die trisubstituierte Silylgruppe zu entfernen.

7. Verfahren nach Anspruch 6, bei dem $R^7$ eine n-Butylgruppe ist und X eine $CH_3COO$-Gruppe ist.

8. Verfahren nach Anspruch 6 oder 7, bei dem die Azetidinon-Verbindung (6) eine Konfiguration der folgenden Formel aufweist :

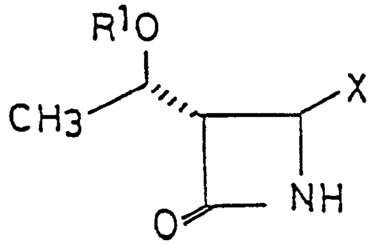

(6)

wodurch die β-Lactam-Verbindung (8) eine Konfiguration der folgenden Formel :

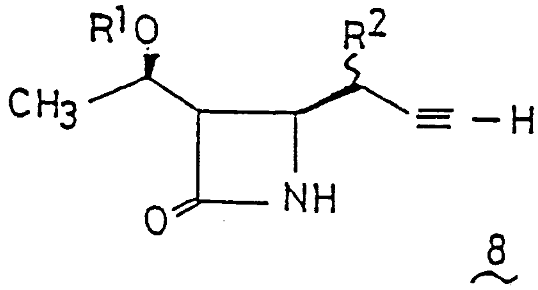

8

aufweist, die β-Lactam-Verbindung (9) eine Konfiguration der folgenden Formel :

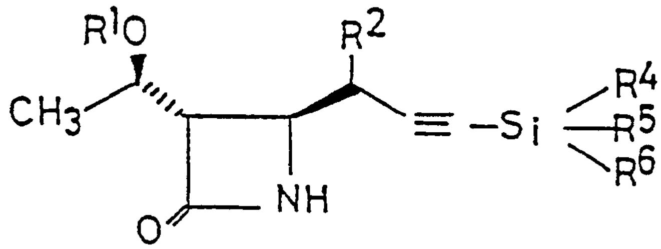 ;

aufweist, und die β-Lactam-Verbindung, die man nach Entfernen der trisubstituierten Silylgruppen erhält, eine Konfiguration der folgenden Formel :

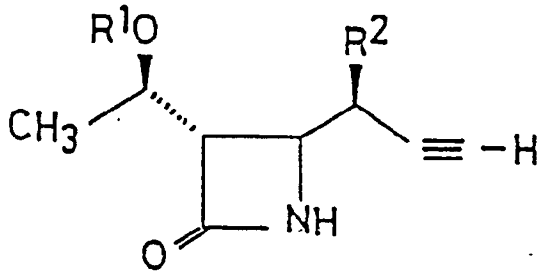

aufweist.

9. Verfahren zur Herstellung eines Azetidinons der Formel :

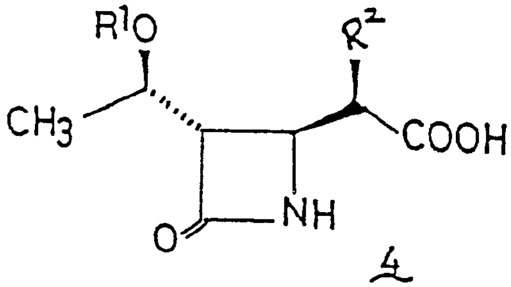

4

(in der $R^1$ und $R^2$ die in Anspruch 1 gegebene Definition aufweisen), bei dem man ein β-Lactam der Formel :

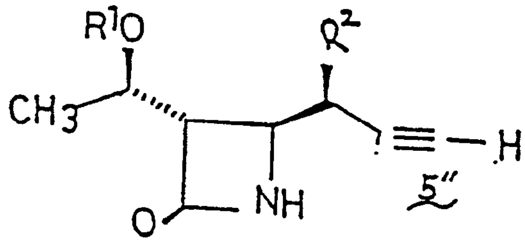

5''

in der $OR^1$ und $R^2$ die in Anspruch 1 gegebene Bedeutung aufweisen, behandelt, um die Ethinylgruppe in eine Carbonsäuregruppe umzuwandeln.